# EUROPÄISCHE PATENTSCHRIFT

(11) **EP 0 800 824 B1**
(45) Veröffentlichungstag und Bekanntmachung des Hinweises auf die Patenterteilung: **22.08.2001**
(21) Anmeldenummer: 97103483.0
(22) Anmeldetag: 04.03.1997
(51) Int. Cl.: A61K 31/07, A61K 9/107, A61K 47/10, A61K 47/24

(54) **Stabile zur parenteralen Verabreichung geeignete Carotinoid-Emulsionen**
Stable carotenoid emulsions for parenteral administration
Emulsions stables de caroténoides pour l'administration parentérale

(30) Priorität: 11.03.1996 DE 19609476
(43) Veröffentlichungstag der Anmeldung: 15.10.1997
(73) Patentinhaber: BASF AKTIENGESELLSCHAFT, 67056 Ludwigshafen (DE)
(72) Erfinder: Schweikert, Loni, Dr., 67122 Altrip (DE); Kolter, Karl, Dr., 67117 Limburgerhof (DE)

(56) Entgegenhaltungen:
- EP-A- 0 100 459
- EP-A- 0 551 638
- DE-A- 4 405 545
- GB-A- 918 399

## Beschreibung

Die Erfindung betrifft Carotiniod-Emulsionen vom Typ Öl-in-Wasser d.h. mit disperser das Carotinoid enthaltender Ölphase, in der die Carotinoidkonzentration höher als die Sättigungskonzentration bei Raumtemperatur ist, sowie ein Verfahren zur Herstellung dieser Emulsionen.

Carotinoide besitzen antioxidative Effekte und sind hervorragende Sauerstoffradikal-Fänger. Die schützende Wirkung der Carotinoide wird von der Natur in Photosynthese-Systemen ausgenutzt. So ist Chlorophyll in grünen Pflanzen mit β-Carotin vergesellschaftet, was eine oxidative Schädigung des Photosynthese-Systems verhindert.

Im Organismus höherer Lebewesen, wie des Menschen, treten infolge von Stoffwechselvorgängen ebenfalls aggressive Sauerstoff-Radikale auf, die für die Entstehung oder Förderung von bestimmten Krankheiten (z.B. Arthritis, Krebs) diskutiert werden.

Carotinoide, insbesondere β-Carotin, kommen in pflanzlichen Lebensmitteln wie Gemüsen oder Salaten vor und werden dem menschlichen Organismus über die Nahrung zugeführt.

Andererseits ist aber bekannt, daß die Resorptionsrate der in der Regel schlecht löslichen Carotinoide sehr gering sein kann. Aus rohen Karotten zum Beispiel wird wenig β-Carotin vom menschlichen Körper aufgenommen. Einseitige Ernährungsgewohnheiten tragen ein übriges dazu bei, daß von bestimmten Bevölkerungsgruppen nur geringe Mengen an Carotinoiden durch die Nahrung aufgenommen werden.

Aus diesem Grunde besteht ein Interesse, Carotinoid-Formulierungen zu entwickeln, die sich zu Injektionszwecken eignen und ein schnelles Ansteigen der Carotinoid-Konzentration im Organismus ermöglichen. Eine besondere Schwierigkeit stellen dabei die allseits bekannten schlechten Löseeigenschaften der meisten Carotinoide dar, die in Wasser vollkommen unlöslich und in Fetten und Ölen nur begrenzt löslich sind.

Die Verwendung von molekulardispersen Carotinoid-Lösungen in Ölen oder öllöslichen Substanzen ist für Injektionszwecke problematisch und für Applikationen direkt in die Blutbahn völlig ungeeignet, da die Gefahr einer sogenannten Fettembolie besteht.

Um schwerlösliche Carotinoide in eine für Injektionszwecke geeignete Form zu überführen, sind mehrere Möglichkeiten gegeben, etwa die Verwendung von Solubilisiermittein, die mit β-Carotin in wäßrigen Systemen Mizellen bilden, oder das Herstellen von β-Carotin-Emulsionen, wobei aufgrund der niedrigen Öllöslichkeit bislang höhere Konzentrationen an β-Carotin in der Regel nicht realisiert werden konnten. Generell muß selbstverständlich auf eine gute physiologische Verträglichkeit aller Komponenten geachtet werden.

Zu diesem Zweck wird in EP-B-O55 817 und EP-A-0 479 066 die Herstellung von Carotinoid-Solubilisaten mit nichtionogenen Emulgatoren, insbesondere vom Typ ethoxilierter Fettsäure beschrieben. Diese mizellaren Lösungen sind zwar prinzipiell zu Injektionszwecken geeignet; jedoch kann die Verwendung ethoxilierter Fettsäuren zu schwerwiegenden Unverträglichkeitsreaktionen (anaphylaktischer Schock) führen.

In DE 2236899 und DE 2254105 werden carotinoidhaltige Emulsionen beschrieben, die bis zu 4 % Carotinoid (Bixin, Crocetin, Xantophyll oder Zeaxanthin) enthalten können. Zur Herstellung dieser Emulsionen sind als Emulgatoren Verbindungen aus Tris-(hydroxymethyl)-aminomethan und C 9-C 20-Fettsäuren sowie als Verdünnungsmittel hohe Konzentrationen eines hydrophilen organischen Lösungsmittels bzw. eines zweiten Emulgators vonnöten. Diese Hilfsstoffe sind in den genannten Konzentrationen schlecht verträglich und erlauben keine parenterale Applikation am Menschen

DE-A-4405545 beschreibt u.a. β-Carotin haltige Emulsionen für Zubereitungen zur oralen Applikation. So werden u.a. Trinkampullen offenbart, deren β-Carotingehalt bei rund 0,075 Gew.-% liegt.

In der japanischen Patentschrift 04026670 wird eine Vitaminzubereitung beschrieben, die ein Carotinoid als Stabilisator enthält. Die Konzentration des Carotinoids ist hierbei auf die Sättigungslöslichkeit in der eingesetzten öllöslichen Substanz (pflanzliche Öle, Kohlenwasserstoffe, Paraffin) beschränkt.

Die Herstellung einer carotinoidhaltigen Fettemulsion bestehend aus β-Carotin oder Zeaxanthin, Sojabohnenöl (10 %), Sojalecithin (1,2 %), Glycerin (2,5 %) und Wasser wird von P. Grolier, V-Azais-Braesco, L. Zelmire und H. Fessi in Biochemica et Biophysica Acta 1111, 135-138 (1992) beschrieben. Als nicht zu überwindendes Hindernis wird von den Autoren die niedrige Öllöslichkeit der genannten Carotinoide angeführt. Obwohl von einer Konzentration von β-Carotin im Öl von 0,12% ausgegangen wurde, wurden nach Herstellung nur Konzentrationen kleiner 0,0037% gefunden. Das bedeutet, daß β-Carotin während der Herstellung ausgefallen ist oder sich zersetzt hat. Mit Zeaxanthin konnten aufgrund der noch niedrigen Öllöslichkeit keine Fettemulsionen hergestellt werden.

Emulsionen mit den Vitaminen A, D, E sind aus dem Tierernährungsgebiet und aus verschiedenen Patentschriften (EP 100 459, JP 581 625 17) bekannt. Da diese Vitamine gut öllöslich sind, ist prinzipiell die Einarbeitbarkeit in eine Emulsion gegeben. Die Schwierigkeiten liegen hierbei in der Herstellung stabiler Zubereitungen, insbesondere wenn parenteral verträgliche Emulgatoren eingesetzt werden sollen und auf Cosolventien wie Glycerin, Propylenglykol oder Polyethylenglykol in höheren Konzentrationen verzichtet werden soll.

Fettemulsionen stellen prinzipiell ein thermodynamisch instabiles System dar, das sehr empfindlich auf Änderungen reagiert. Schon leichte Veränderungen in Art und Menge von Wirkstoffen oder Hilfsstoffen können zur Instabilität des gesamten Systems führen (J. Pharm. Sci, 82 (11), 1069-78 (1993). Dies ist deshalb von ausschlaggebender Bedeutung, weil an Fettemulsionen zur parenteralen Applikation besondere Anforderungen gestellt werden müssen, um das Leben der Patienten nicht in Gefahr zu bringen. Besonders wichtig sind in diesem Zusammenhang die Teilchengröße - Teilchen größer 1 µm können zur Fettembolie führen - die systemische und lokale Verträglichkeit und die hämolytische Aktivität. Aus Verträglichkeitsgründen muß eine parenterale Fettemulsion annähernd die Osmolarität des Blutes ca. 300 mosmol aufweisen. Liegt die Osmolarität niedriger, platzen die Blutkörperchen bei der Verabreichung, liegt sie höher wird ihnen Wasser entzogen, wodurch sie schrumpfen. Beides führt zur Schädigung. Diese Tatsache verbietet zugleich die parenterale Anwendung der für orale Applikation bekannten Emulsionen, die große Mengen Polyole, wie Glycerin enthalten, am Menschen.

In den PCT-Anmeldungen WO 94/21231 und WO 94/21232 werden β-Carotin-haltige Emulsionen mit einem hohen Glycerinanteil (30 bis 90 %) beschrieben. Diese Zubereitungen erfüllen wegen der hohen Konzentration an Glycerin das Erfordernis der Blutisotonie nicht und sind deswegen bei parenteraler Anwendung unverdünnt nicht verträglich. Ein Herunterverdünnen hat andererseits die Nachteile, daß das Volumen der Injektionsflüssigkeit zunimmt und sich die Emulsion durch die Verdünnung verändern kann, z.B. instabil wird oder die Tröpfchengröße unkontrolliert ansteigt. Dies ist aus bereits geschilderten Gründen (Gefahr der Fettembolie) nicht akzeptabel.

Daher bestand die Aufgabe Systeme zu entwickeln, die gut verträgliche Dispergiermittel enthalten, hohe Konzentrationen von Carotinoiden ermöglichen, lagerstabil sind und das Erfordernis der Blutisotonie erfüllen.

Diese Aufgabe wurde erfindungsgemäß gelöst mit stabilen, zur parenteralen Verabreichung geeigneten Emulsionen vom Typ Öl-in-Wasser, bestehend aus einer Wasserphase und einer mittels eines physiologisch verträglichen Emulgators feinstverteilten dispersen Ölphase mit Teilchengrößen kleiner 1 µm, die mindestens ein Carotinoid enthält und auf einem physiologisch verträglichen Öl oder Fett basiert, wobei die Emulsion weiniger als 10 Gew.-% Glycerin und als Emulgator Polyoxyethylen-Polyoxypropylen-Blockpolymere und/oder Lecithin enthält und das Carotinoid in der Ölphase bei einer Temperatur von 120°C bis 250 °C gelöst wird.

Bei der vorliegenden Erfindung kann man im Prinzip ganz auf Glycerin verzichten bzw. die Glycerinkonzentration kann weniger als 10 Gew.-% betragen. Vorzugsweise liegt die Konzentration des Glycerins zwischen 1 und 3 Gew.-%, zum Einstellen der Blutisotonie.

Träger für die Carotinoide in der Ölphase sind für Injektionszwecke zugelassene Öle, wie sie in dem Lehrbuch Pharmazeutische Technologie, herausgegeben von H. Sucker, P. Fuchs und P. Speiser, 2. Auflage (1991), S. 459 und 504 aufgelistet sind, z.B. Sojaöl oder mittelkettige Triglyceride, wie sie in Kokosöl vorliegen. Zudem wurde gefunden, daß Vitamin E oder A oder deren Derivate hervorragend als Träger für Carotinoide verwendet werden können, vor allem wenn es das Ziel ist, vitaminhaltige Carotinoid-Emulsionen herzustellen.

Insbesondere eignet sich als Trägeröl das ebenfalls als Antioxidans wirkende Vitamin E (Tocopherol) bzw. ölige Derivate davon, wie das Tocopherolacetat. Deren Verwendung bietet den Vorteil, daß eine von eßbaren Fetten und Ölen völlig freie Zubereitung hergestellt werden kann und somit keine negativen Auswirkungen eines Fettanteiles (Erhöhung des Triglyceridwertes im Blut etc.) zu befürchten sind.

Als Carotinoide kommen alle in der Hitze in Öl löslichen Carotinoide in Betracht, z.B. Canthaxanthin, Citranaxanthin, Zeaxanthin, Apocarotinal, Apocarotinsäure-Derivate wie Apocarotinsäureethylester, sowie Gemische davon. Für einen Einsatz als Antioxidans bzw. Radikalfänger eignen sich aber insbesondere β-Carotin, Lycopin, Astaxanthin oder ein Gemisch davon.

Auch lassen sich der wäßrigen Phase weitere wasserlösliche Wirkstoffe zusetzen, wie wasserlösliche Vitamine und Spurenelemente. Insbesondere Vitamin C oder Natriumascorbat, die ebenfalls als Antioxidans im Organismus wirken, kommen als Zusatz in Betracht. Zur Erhöhung der mikrobiologischen Stabilität kann es von Vorteil sein, der Injektionsemulsion begrenzte Mengen (max. 10 %) an Ethylalkohol oder andere verträgliche Konservierungsmittel wie Thimerosal oder Chlorkresol etc. zuzusetzen.

Als Emulgator kommen an sich bekannte physiologisch verträgliche Dispergiermittel aus der Gruppe der Propylenoxid-Ethylenoxid-Blockpolymere oder der Gruppe der Lecithine sowie Mischungen von beiden Gruppen in Betracht, wobei die Polyoxyethylen-Polyoxypropylen-Blockpolymere bevorzugt sind. Die erfindungsgemäß zu verwendenden Polyoxyethylen-polyoxypropylen-Blockpolymerisate - auch Poloxamere genannt - sind Verbindungen der Formel 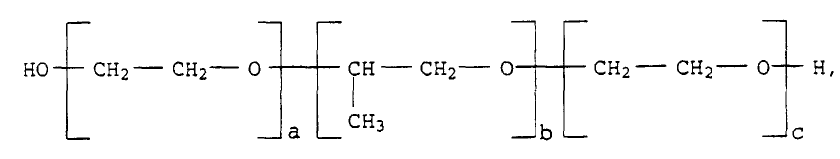 in der a und c 2 bis 130 und b 15 bis 67 Einheiten bedeutet. Insbesondere kommt ein Produkt in Betracht, bei dem a und c ca. 80 und b ca. 30 beträgt. Diese Emulgatoren können durch sogenannte Coemulgatoren wie z.B. Mono- oder Diglyceride, Ascorbylfettsäureester oder Fettsäuren, Fettsäuresalze in ihrer Wirkung verstärkt werden.

Die Löslichkeit der meisten Carotinoide in Ölen ist so gering, daß bei Raumtemperatur 0,05-0,1 % kaum überschritten werden. Daher wird das Carotinoid durch kurzzeitiges Erhitzen über 120°C in dem Träger gelöst, wodurch die Löslichkeit beträchtlich gesteigert wird. Die Konzentration des Carotinoids in dem öligen Träger kann dann 0,2 % und 50 Gew.-% betragen und liegt vorzugsweise zwischen 10 % und 20 Gew.-%.

Ausgehend von diesen Carotinoid/Öl- bzw. CarotinoidNitamin-Gemischen lassen sich Öl-in-Wasser Emulsionen herstellen, die einen Carotinoid-Gehalt von mindestens 0,1 % aufweisen. Die erzielbare Obergrenze der Carotinoid-Konzentration in der Emulsion wird nicht zuletzt durch den Gesamtanteil an dispergierter Phase in der Injektionsemulsion begrenzt, der typischerweise 50 % nicht überschreiten sollte. Daraus resultiert eine maximale erzielbare Carotinoid-Konzentration von 25 % in der fertigen Injektionsemulsion. In der Regel wird der Carotinoidgehalt, bezogen auf die Emulsion 10 Gew.-% nicht überschreiten.

Demgemäß enthalten die erfindungsgemäßen Zubereitungen, bezogen auf die fertige Emulsion,
0,1 bis 10, vorzugsweise 0,1 bis 5 Gew.-% Carotinoid, vorzugsweise β-Carotin,
0,2 bis 50, vorzugsweise 1 bis 20 Gew.-% öliger Träger, 0,1 bis 15 Gew.-%, vorzugsweise 1,0 bis 10 Gew.-% Emulgator und der Rest Wasserphase, die gegebenenfalls 0,001 bis 1 Gew.-% Mineralstoffe wie Cu-, Se-, Zn- oder Mn-Salze und gewünschtenfalls weitere wasserlösliche Vitamine wie z.B. Vitamin C und die Vitamine B₁, B₂, B₆, B₁₂ enthält.

Die erfindungsgemäßen Injektionsemulsionen können auch noch zusätzlich an sich bekannte Antioxidantien wie Butylhydroxitoluol, Butylhydroxianisol, Tocopherol oder Ascorbylpalmitat in üblichen Mengen, d.h. 0,01 bis 2 Gew.-% zur weiteren Erhöhung der Stabilität z.B. des Trägeröls enthalten.

Überraschenderweise sind solche übersättigten Carotinoidemulsionen über lange Zeit stabil, d.h. es erfolgt keine Rekristallisation des Wirkstoffes oder Zusammenfließen von Emulsionströpfchen. Wie alle Injektions-Emulsionen müssen die erfindungsgemäßen Emulsionen einen hohen Feinheitsgrad aufweisen (keine Partikel größer als 1 µm). Zur Herstellung der Emulsionen können die an sich bekannten Technologien verwendet werden, die zur Herstellung einer Emulsion mit dem geforderten Feinheitsgrad geeignet sind.

Allgemein gilt, daß die Erhitzung des Carotinoids auf z.B. 120 bis 250°C im Öl möglichst kurz sein soll und möglichst schnell nach vollständiger Lösung auf Raumtemperatur oder nahe Raumtemperatur abgekühlt werden sollte.

Dies geschieht in der Regel durch Einemulgieren der heißen Öllösung in die Wasserphase, die bei Raumtemperatur oder leicht erhöhter Temperatur vorgelegt wird.

Man kann das Emulgieren kontinuierlich oder diskontinuierlich durchführen.

Bei der diskontinuierlichen Arbeitsweise wird z.B. Wasser für Injektionszwecke, das das Dispergiermittel und gegebenenfalls physiologisch verträgliche Alkohole wie z.B. Sorbit, Xylit oder Glycerin enthält, vorgelegt, wobei die Temperatur der vorgelegten Wasserphase auf Raumtemperatur bis leicht erhöhter Temperatur z.B. bis 50°C gehalten wird. In die Wasserphase wird die Carotinoid/Öllösung mit einem Hochgeschwindigkeitsrührer z.B. mit einem Ultraturrax® eingerührt und anschließend die erhaltene Emulsion mit einem Hochdruckhomogenisator homogenisiert.

Bei der kontinuierlichen und bevorzugten Arbeitsweise geht man analog der Methodik vor, wie sie in DE-A-37 02 030 beschrieben ist und auf die bezüglich der apparativen Technik und der Verfahrensdurchführung ausdrücklich Bezug genommen wird und deren Angaben als in die vorliegende Beschreibung mit der Maßgabe der im folgenden beschriebenen Abänderung integriert gelten sollen.

Wesentlicher Bestandteil dieses Verfahrens sind zwei Mischkammern. Die erste Mischkammer dient dazu, daß man das Carotinoid in einem wassermischbaren organischen Lösungsmittel zusammen mit einem eßbaren Öl, sowie einem Emulgator, gegebenenfalls unter Druck, bei Temperaturen zwischen 50°C und 240°C rasch löst, die erhaltene molekulardisperse Lösung in der zweiten Mischkammer sofort mit einer wäßrigen Lösung eines Schutzkolloids intensiv vermischt, wobei das Lösungsmittel in die wäßrige Phase überführt wird und die hydrophobe Ölphase, die das Carotinoid gelöst enthält als mikrodisperse Phase entsteht. Die erhaltene Zweiphasen-Mischung wird dann vom Lösemittel und Wasser befreit, so daß eine pulverförmige Zubereitung entsteht.

Dieses Verfahren wird zur Herstellung der erfindungsgemäßen Injektionslösung dergestalt abgewandelt, daß eine kolloidfreie Wasserphase, in der zweckmäßig der Emulgator enthalten ist, mit einer heißen Lösung des Carotinoids in der Mischung aus Öl und wassermischbaren organischen Lösungsmittel turbulent gemischt wird. Sofern erforderlich kann die Emulsion anschließend mit einem Hochdruckhomogenisator homogenisiert werden. Die Menge des wassermischbaren Lösemittels in der ersten Mischkammer, vorzugsweise Ethylalkohol wird dabei so gewählt, daß sie in der erhaltenen Injektionslösung, gegebenenfalls auch nach Verdünnung, eine in fertigen Injektionslösungen physiologisch verträgliche Konzentration von z.B. 10 Gew.-% nicht überschreitet. Weitere in Injektionslösungen übliche wasserlösliche Bestandteile wie pH-Regulatoren, Isotonisierungsmittel, Salze oder andere Wirkstoffe wie Vitamin C werden entweder vor Herstellung der Emulsion der Wasserphase beigemischt oder nach Fertigstellung der Emulsion zugegeben.

Anstelle der Herstellung der Carotinoidlösung in der ersten Mischkammer ist auch die kontinuierliche Durchleitung einer Carotinoid/Ölsuspension durch einen Wärmetauscher und weitere Verarbeitung wie oben beschrieben möglich.

Die Abfüllung der Carotinoidemulsion erfolgt in bekannter Weise unter sterilen Bedingungen in Infusionsflaschen, Vials, Ampullen oder Fertigspritzen. Sofern erforderlich können diese einem schonenden thermischen Keimreduzierungsverfahren unterworfen werden.

Die hochkonzentrierte Carotinoid-Emulsionen können direkt parenteral verabreicht werden oder vor Applikation mit kompatiblen wäßrigen Infusionslösungen oder Fettemulsionen zur parenteralen Ernährung verdünnt werden.

Neben der parenteralen Applikation ist auch eine orale oder dermale Anwendung möglich, wobei infolge der Übersättigung mit β-Carotin eine hohe Resorption durch die Schleimhaut bzw. Haut zu erwarten ist.

Bisher konnten sogenannte Antioxidanszubereitungen, die β-Carotin, Tocopherol bzw. Tocopherolacetat und Ascorbinsäure enthalten nur für die orale Applikation in Tabletten- oder Kapselform zur Verfügung gestellt werden. Diesen Antioxidanszubereitungen werden zahlreiche Wirkungen wie Prävention von Herzinfarkt, Stroke, Krebs, Maculadegeneration, Parkinson, Alzheimersche Erkrankung etc. zugeschrieben. Mit der vorliegenden Erfindung können diese Stoffe in einer Zubereitung entweder direkt oder auch nach Verdünnung parenteral appliziert werden. Doch nicht nur Antioxidanszubereitungen, sondern auch β-carotinhaltige Multivitamininjektions- oder Infusionslösungen können in analoger Weise hergestellt werden.

In den folgenden Beispielen wurde der Emulgator der wäßrigen Phase zugesetzt. Prinzipiell ist aber auch die Zugabe zur lipophilen Phase möglich, insbesondere wenn sich der Emulgator darin löst.

### Beispiel 1:

In einem 1 I Becherglas werden 418 ml demineralisiertes Wasser (Wasser für Injektionszwecke) gemischt mit 40 g Propylenoxid-Ethylenoxid-Blockpolymer (Lutrol® F 68 der Firma BASF, Poloxamer 188) und 10 g Glycerin und in einem Wasserbad auf 45°C erwärmt. In einem 100 ml Rundkolben werden 32 g einer 30 %igen β-Carotin Dispersion in fraktioniertem Cocosöl (Miglyol Öl 810) mittels eines Ölbades und unter Rühren kurzzeitig auf 180°C erhitzt, wobei das β-Carotin in Lösung geht.

Diese ölige Lösung wird dann in die wäßrige Phase einemulgiert. Die Emulgierung erfolgt bei einer Temperatur von 45°C mittels eines Ultraturrax® (7000-8000 U/min.) über einen Zeitraum von 8 min. Danach wird die Emulsion bei 1000 bar hochdruckhomogenisiert. Die fertige Emulsion, die einen β-Carotin-Gehalt von 1,6 % und eine mittlere Partikelgröße von 210 nm aufweist, wird mit 0,1 % Thiomersal konserviert und in 10 ml-Vials abgefüllt.

### Beispiel 2:

Mit der Technik der Mischkammermikronisierung gemäß den Angaben der DE-A 37 02 030 wird zunächst in der ersten Mischzelle eine 4,4 %ige ethanolische β-Carotin Dispersion, die 20 % Tocopherol enthält, mit 220°C heißem Ethanol unter Druck (Einstellung des Druckbegrenzungsventils auf 35 bar) gemischt, wobei sich unter den gegebenen Bedingungen in der Mischzelle eine Temperatur von 190°C einstellt. Dabei geht das β-Carotin in Lösung. Die Mengenströme betragen 2 kg/h an wirkstoffhaltiger Dispersion und 2,5 kg/h an reinem Ethanol.

Diese heiße Lösung wird dann in der nachgeschalteten zweiten Mischzelle mit der wäßrigen Phase, die mit einer Pumprate von 27 kg/h zudosiert wird, turbulent gemischt. Die Wasserphase enthält neben entmineralisiertem Wasser (Wasser für Injektionszwecke) 3,4 % Ascorbinsäure, 0,6 % Propylenoxid-Ethylenoxid-Blockpolymer (Lutrol F 68 = Poloxamer 188) und der pH wurde mit 1 m NaOH auf 7,5 eingestellt.

Beim Abmischen in der zweiten Mischkammer entstehen mikrodisperse Tröpfchen, die β-Carotin und Tocopherol enthalten und eine mittlere Teilchengröße von 210 nm aufweisen. Die erhaltene Emulsion enthält 15 % Ethanol und wird, um zu einer verträglichen Injektionszubereitung zu gelangen mit entmineralisiertem Wasser verdünnt, so daß ein Ethanolgehalt von 8 % erreicht wird. Der β-Carotin-Gehalt in der verdünnten Dispersion beträgt 0,15 %, der Gehalt an Tocopherol 0,68 % und der Gehalt an Ascorbinsäure 1,58 %.

### Beispiel 3:

14 g Glycerin, 35 g Lutrol® F 68 (BASF Aktiengesellschaft), 35 g Ascorbylpalmitat und 19,67 g Natriumascorbat werden bei 60°C in 550,8 g Wasser für Injektionszwecke gelöst. Der pH-Wert der Lösung wird mit NaOH auf 7,4 eingestellt. In einem Rundkolben wird eine Mischung aus 56 g fraktioniertem Cocosöl (Miglycol 812) und 14 g Tocoperholacetat auf 180°C erhitzt, unter Stickstoff mit 7 g β-Carotin versetzt und die Mischung bis zum vollständigen Auflösen des β-Carotins gerührt. Die erhaltene β-Carotin-Lösung wird in die wäßrige Phase mit Hilfe eines Ultra-Turrax® bei 3000 U/min einemulgiert. Diese Voremulsion wird anschließend zweimal über einen Hochdruckhomogenisator bei 1000 bar weiter emulgiert und in Vials abgefüllt. Der Gehalt an β-Carotin beträgt 1,0 Gew.-%, an Tocopherolacetat 2,0 Gew.-% und an Natriumascorbat 2,8 Gew.-%. Die mittlere Teilchengröße liegt bei 200 nm. Die Konzentration des β-Carotins bezogen auf die Ölphase beträgt 10 Gew.-%.

### Beispiel 4:

Die Herstellung der Emulsion erfolgt wie im Beispiel 3 beschrieben, wobei jedoch Miglycol 812 durch Tocopherolacetat ersetzt und kein Natriumascorbat verwendet wird. Somit besteht die lipophile Phase gänzlich aus Tocopherolacetat. Der Gehalt, bezogen auf die Gesamtemulsion an β-Carotin beträgt 1,0 Gew.-%, an Tocopherolacetat 10,0 Gew.-%. Die Teilchengröße liegt bei 250 nm. Die Konzentration des β-Carotins in der Ölphase beträgt 10 Gew.-%.

### Beispiel 5:

14 g Glycerin, 35 g Lutrol F68, 3,5 g Ascorbylpalmitat, 7,0 g Natriumascorbat, 0,7 g Riboflavin-5-phosphat-Natrium x 2 H₂O, 0,7 g Thiaminhydrochlorid, 2,1 g Nicotinamid und 0,7 g Pyridoxinhydrochlorid werden bei 60°C in 562,8 g Wasser für Injektionszwecke gelöst. Der pH-Wert wird mit 1-molarer NaOH auf 7,4 eingestellt. In einem Rundkolben wird eine Mischung aus 66,5 g fraktioniertem Cocosöl (Miglycol 812) und 3,5 Tocopherolacetat auf 180°C erhitzt, unter Stickstoff mit 3,5 g β-Carotin versetzt und die Mischung bis zum vollständigen Auflösen des β-Carotins gerührt. Die ölige Lösung wird mit Hilfe eines Ultra-Turrax® bei 3000 U/min in die wäßrige Lösung der Vitamine einemulgiert. Die weitere Emulgierung zu einer feinteiligen Emulsion erfolgt durch 2 Passagen über einen Hochdruckhomogenisator bei 1000 bar. Anschließend wird die Emulsion auf Raumtemperatur abgekühlt und in Vials abgefüllt.

Gehalt, bezogen auf die Gesamtemulsion,

| | | |
|---|---|---|
| an | β-Carotin: | 0,5 Gew.-% |
| | Tocopherolacetat: | 0,5 Gew.-% |
| | Natriumascorbat: | 1,0 Gew.-% |
| | Riboflavin-5-phosphat-Na: | 0,1 Gew.-% |
| | Thiamin-HCl: | 0,1 Gew.-% |
| | Nicotiamid: | 0,3 Gew.-% |
| | Pyridoxin HCI: | 0,1 Gew.-% |

Die Teilchengröße liegt bei 200 nm. Die Konzentration des β-Carotins bezogen auf die Ölphase beträgt 5 Gew.-%.

## Patentansprüche

1. Stabile, zur parenteralen Verabreichung geeignete Emulsion vom Typ Öl-in-Wasser, bestehend aus einer Wasserphase und mittels eines physiologisch verträglichen Emulgators feinstverteilten dispersen Ölphase mit Teilchengrößen kleiner 1 µm, die mindestens ein Carotinoid enthält und auf einem physiologisch verträglichen Öl oder Fett basiert, dadurch gekennzeichnet, daß die Emulsion weniger als 10 Gew.-% Glycerin und als Emulgator Polyoxyethylen-Polyoxypropylen-Blockpolymere und/oder Lecithin enthält und daß das Carotinoid in der Ölphase bei einer Temperatur von 120°C bis 250°C gelöst wird.

2. Stabile Emulsion gemäß Anspruch 1, dadurch gekennzeichnet, daß sie 1 bis 3 Gew.-% Glycerin enthält.

3. Stabile Emulsion gemäß Anspruch 1, dadurch gekennzeichnet, daß die Emulsion als Coemulgator Mono- oder Diglyceride, Ascorbylfettsäureester oder Fettsäuren bzw. Fettsäuresalze enthält.

4. Stabile Emulsion gemäß Anspruch 1, dadurch gekennzeichnet, daß das Öl der Ölphase Sojaöl, ein mittelkettiges Triglycerid, Tocopherol oder Tocopherolacetat ist.

5. Stabile Emulsion gemäß Anspruch 1, dadurch gekennzeichnet, daß das Carotinoid Beta-Carotin ist.

6. Stabile Emulsion gemäß Anspruch 1, dadurch gekennzeichnet, daß die Ölphase Beta-Carotin und Tocopherol oder Tocopherolacetat und die Wasserphase Ascorbinsäure oder Natriumascorbat enthält.

7. Stabile Emulsion gemäß Anspruch 1, dadurch gekennzeichnet, daß die Ölphase weitere öllösliche Vitamine und die Wasserphase weitere wasserlösliche Vitamine sowie Spurenelemente enthält.

8. Stabile Emulsion gemäß Anspruch 1, dadurch gekennzeichnet, daß, bezogen auf die gesamte Emulsion, 0,1 bis 10 Gew.-% Carotinoid und 0,2 bis 50 Gew.-% Öl oder Fett enthalten sind.

9. Stabile Emulsion gemäß Anspruch 1, dadurch gekennzeichnet, daß sie als Emulgator Polyoxyethylenpolyoxypropylen-Blockpolymere enthält.

10. Verfahren zur Herstellung der zur parenteralen Verabreichung geeigneten Emulsionen gemäß Anspruch 1, dadurch gekennzeichnet, daß man eine Suspension eines Carotinoids in einem physiologisch verträglichen Öl oder Fett-wobei das Carotinoid in einer über der der Sättigungslöslichkeit bei Raumtemperatur entsprechenden Menge vorliegt-durch kurzzeitiges Erhitzen auf Temperaturen von 120 bis 250°C in Lösung bringt und die Lösung mittels eines Emulgators mit Wasser emulgiert.

## Claims

1. A stable oil-in-water emulsion suitable for parenteral administration, consisting of an aqueous phase and of an oil phase which is very finely dispersed by means of a physiologically tolerated emulsifier and has particle sizes below 1 µm, and which contains at least one carotenoid and is based on a physiologically tolerated oil or fat, wherein the emulsion comprises less than 10 % by weight glycerol and comprises as emulsifier polyoxyethylene/polyoxypropylene block copolymers and/or lecithin, and wherein the carotenoid is dissolved in the oil phase at from 120 to 250°C.

2. A stable emulsion as claimed in claim 1, which comprises from 1 to 3 % by weight glycerol.

3. A stable emulsion as claimed in claim 1, which comprises as coemulsifier mono- or diglycerides, ascorbyl fatty acid esters or fatty acids or fatty acid salts.

4. A stable emulsion as claimed in claim 1, wherein the oil in the oil phase is soybean oil, a medium chain-length triglyceride, tocopherol or tocopherol acetate.

5. A stable emulsion as claimed in claim 1, wherein the carotenoid is beta-carotene.

6. A stable emulsion as claimed in claim 1, wherein the oil phase comprises beta-carotene and tocopherol or tocopherol acetate and the aqueous phase comprises ascorbic acid or sodium ascorbate.

7. A stable emulsion as claimed in claim 1, wherein the oil phase comprises other oil-soluble vitamins and the aqueous phase comprises other water-soluble vitamins and trace elements.

8. A stable emulsion as claimed in claim 1, which comprises from 0.1 to 10 % by weight carotenoid and from 0.2 to 50 % by weight of oil or fat, based on the complete emulsion.

9. A stable emulsion as claimed in claim 1, which comprises as emulsifier polyoxyethylene/polyoxypropylene block copolymers.

10. A process for the preparation of the emulsions suitable for parenteral administration as claimed in claim 1, which comprises dissolving a suspension of a carotenoid in a physiologically tolerated oil or fat, the carotenoid being in an amount corresponding to more than the saturation solubility at room temperature, but briefly heating at from 120 to 250°C, and emulsifying the solution with water by means of an emulsifier.

## Revendications

1. Emulsion stable, convenant pour l'administration parentérale, du type huile-dans-l'eau, consistant en une phase aqueuse et une phase huileuse, en dispersion extrêmement fine grâce à un agent émulsionnant physiologiquement acceptable, ayant une dimension de particule inférieure à 1 µm, qui contient au moins un caroténoide et est à base d'une huile ou matière grasse physiologiquement acceptable, caractérisée par le fait que l'émulsion contient moins de 10 % en poids de glycérol et contient en tant qu'agent émulsionnant des polymères séquencés polyoxyéthylène-polyoxypropylène et/ou de la lécithine, et que le caroténoide est dissous dans la phase huileuse à une température de 120 à 250°C.

2. Emulsion stable selon la revendication 1, caractérisée par le fait qu'elle contient 1 à 3 % en poids de glycérol.

3. Emulsion stable selon la revendication 1, caractérisée par le fait que l'émulsion contient comme agent émulsionnant des mono- ou diglycérides, des esters d'acide gras d'ascorbyle ou des acides gras ou respectivement des sels d'acides gras.

4. Emulsion stable selon la revendication 1, caractérisée par le fait que l'huile de la phase huileuse est de l'huile de soja, un triglycéride à chaînes moyennes, du tocophérol ou de l'acétate de tocophérol.

5. Emulsion stable selon la revendication 1, caractérisée par le fait que le caroténoide est le bêta-carotène.

6. Emulsion stable selon la revendication 1, caractérisée par le fait que la phase huileuse contient du bêta-carotène et du tocophérol ou de l'acétate de tocophérol et la phase aqueuse de l'acide ascorbique ou de l'ascorbate de sodium.

7. Emulsion stable selon la revendication 1, caractérisée par le fait que la phase huileuse contient d'autres vitamines solubles dans l'huile et la phase aqueuse contient d'autres vitamines hydrosolubles, ainsi que des oligo-éléments.

8. Emulsion stable selon la revendication 1, caractérisée par le fait que, sur la base de l'ensemble de l'émulsion, 0,1 à 10 % en poids, de caroténoide et 0,2 à 50 % en poids d'huile ou de matière grasse y sont contenus.

9. Emulsion stable selon la revendication 1, caractérisée par le fait qu'elle contient comme agent émulsionnant des polymères séquencés de polyoxyéthylène-polyoxypropylène.

10. Procédé pour la préparation d'émulsions appropriées pour administration parentérale selon la revendication 1, caractérisé par le fait qu'on met en solution une suspension d'un caroténoide dans une huile ou matière grasse physiologiquement acceptable - tandis que le caroténoïde est présent en une quantité correspondante supérieure à la solubilité à saturation à température ambiante - par chauffage bref à des températures de 120 à 250°C et on émulsionne la solution avec de l'eau, au moyen d'un agent émulsionnant.
